# EUROPEAN PATENT APPLICATION

(11) **EP 3 613 855 A1**
(43) Date of publication of application: **26.02.2020**
(21) Application number: 18190452.5
(22) Date of filing: 23.08.2018
(51) Int. Cl.: C12N 15/10

(54) **METHOD FOR THE PRODUCTION OF A NUCLEIC ACID LIBRARY**

(71) Applicant: Clariant Produkte (Deutschland) GmbH, 65929 Frankfurt am Main (DE)
(72) Inventor: JOCHENS, Helge, 82065 Baierbrunn (DE); SCHATTE, Martin, 81479 München (DE)
(74) Representative: Graser, Konstanze

(57) **Abstract**

The present invention refers to a method for the production of a nucleic acid library comprising *in silico* and *in vitro* and/or *in vivo* method steps. In particular, the method employs fragmentation in silico and synthesis of oligonucleotides in chambers from which they can easily be brought into contact for reassemby. The invention is further directed to a method for detecting a disease based on the nucleic acid library.

## Description

The present invention refers to a method for the production of a nucleic acid library, where the selection of nucleic acid sequences and the fragmentation of the selected nucleic acid sequences are performed *in silico* and the production of oligonucleotides, the reassembling of the oligonucleotides, their optional amplification and optional screening *in vitro* and/or *in vivo* for the reassembled oligonucleotide of interest are performed *in vitro* and/or *in vivo* The invention is further directed to a method for detecting a disease based on the nucleic acid library.

### Technical background

DNA shuffling is an established recombinatorial method that was originally developed to increase the speed of directed evolution experiments beyond what could be accomplished using error-prone PCR alone (Stemmer WPC, Proc. Nat. Acad. Sci., 1994, 10747-10751). To achieve this, mutated copies of a protein-coding sequence are fragmented with DNase I and the fragments are then reassembled in a PCR without primers. The fragments anneal where there is sufficient sequence identity, resulting in full-length variants of the original gene that have inherited mutations from multiple templates. Subsequent studies demonstrated that directed evolution could be further accelerated by shuffling similar native protein-coding sequences from the same gene family, rather than mutated variants of a single gene. Since DNA shuffling was originally developed, many variations of the method have been published. In particular, the use of restriction enzymes in the fragmentation step allows for greater customization of fragment lengths than DNase I digestion and avoids the risk that parental sequences may be over-digested into unusable very small fragments. Restriction enzyme-mediated fragmentation also reduces the occurrence of undigested parental sequences that would otherwise reduce the number of unique variants in the resulting library (Behrendorff JB et al., Methods Mol. Biol., 2014, 1175-1187).

Family shuffling utilizes naturally occurring nucleotide substitutions among family genes as the driving force for the *in vitro* evolution. The application of the family shuffling strategy has also provided many successful examples. Contemporary genes belonging to the same gene family are derived from a single ancestral gene after repeated introduction of mutations through the natural divergent evolution processes. The shuffling of the family gene sequences creates a library of chimeric genes, from which desired chimera are selected.

Thus, methods for the recombination of nucleic acid sequences such as genes are majorly used to combine beneficial mutations found in different nucleic acid sequences into one nucleic acid sequence such as different genes into one gene.

In one very common approach, random mutagenesis is used to introduce mutations into a nucleic acid, e.g., a gene of interest, followed by screening for the most potent mutants. As the mutants found usually contain more than one mutation and it is usually not obvious which mutation is actually the one that is beneficial, random recombination and screening is used to combine beneficial mutations into one nucleic acid sequence, such as a gene of interest for example based on DNA shuffling (Stemmer WPC, Proc. Nat. Acad. Sci., 1994, 10747-10751).

In another approach, homologous nucleic acid sequences such as genes are recombined to gain new variants that contain the beneficial variance of related proteins (Family Shuffling).

In both technologies, one has to get the nucleic acid sequences for example genes to be recombined physically in hands in order to recombine them. That means for example that for a family shuffling approach in which for example 100 homologous genes are recombined, the 100 genes have to be synthesized first which is extremely time consuming and cost-intensive. Afterwards, the genes are randomly fragmented by enzyme treatment to create oligonucleotides, which bears quite some limitations in the fragmentation of the genes and thus, of the creation of the oligonucleotides. These oligonucleotides are then re-assembled to create new full-length genes.

The method of the present invention allows to create nucleic acid sequence libraries such as gene libraries from homologous genes without having the nucleic acid sequences in hands beforehand. The selection and fragmentation of the nucleic acid sequences such as a gene is done virtually, i.e., *in silico. In silico* is defined as performed on computer or via computer simulation. The oligonucleotides are synthesized *in vitro* and eluted in the same vessel resulting in a pool of for example hundreds or thousands of individual oligonucleotides. In an assembly reaction, the nucleic acid sequences such as full length genes used as starting point for the present method are re-assembled and due to the sequence identity and thus, homologous regions a library containing variants in nucleic acids within the complete nucleic acid sequence are generated that are identified by selection and screening, respectively. The synthesizing of oligonucleotides in very high numbers in a relatively short period of time and for a very reasonable price without previous synthesizing and fragmenting the starting nucleic acid sequence makes the method of the present invention highly advantageous compared to the methods of the prior art.

In addition, the fragmentation of the oligonucleotide is either performed randomly, or controlled, e.g., by synthesizing an oligonucleotide per nucleotide of the nucleic acid sequence just by walking along the nucleic acid sequence in 5' to 3' or 3' to 5' direction or synthesizing an oligonucleotide based on regions of the nucleic acid sequence encoding protein structures such as a helix or beta-sheet etc., or by combinations thereof.

### Description of figures

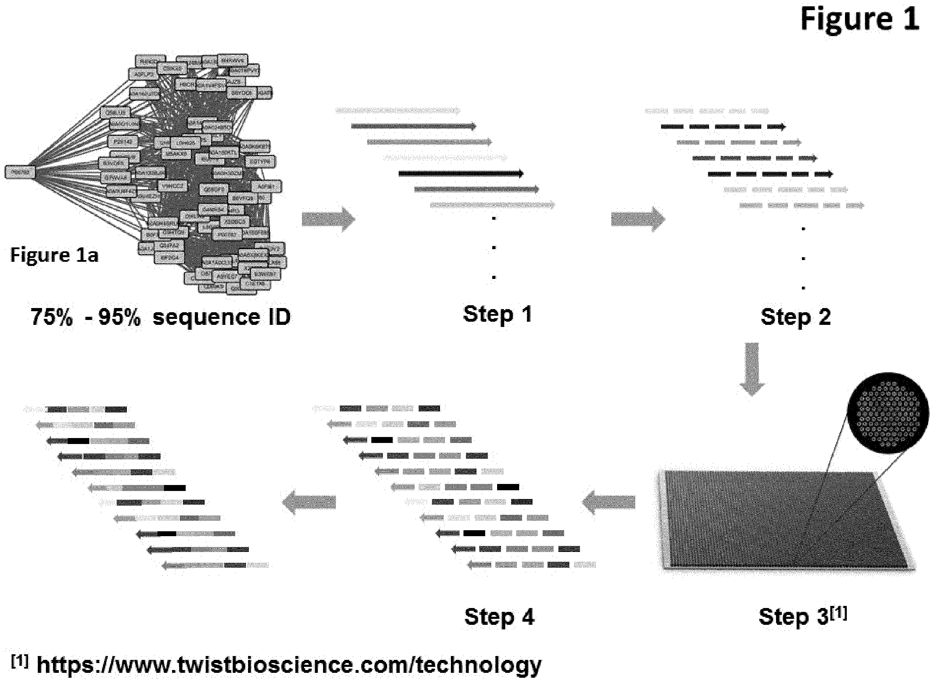

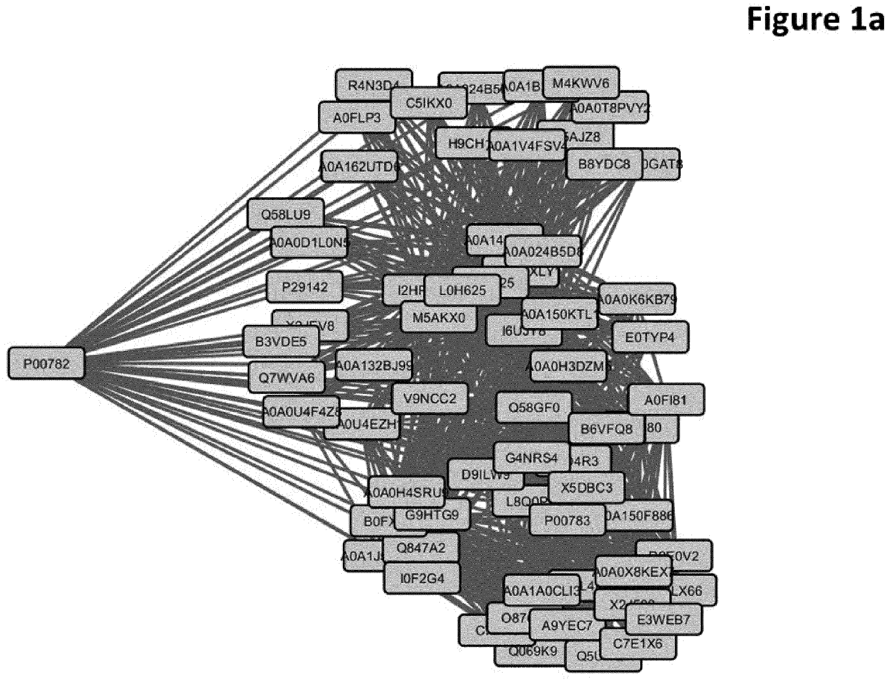

Fig. 1 shows the principle of the method of the present invention for producing a nucleic acid library, where nucleic acid sequences are fragmented into an oligonucleotide pool, which is then reassembled corresponding to the starting nucleic acid sequences, wherein one or more nucleotides have been exchanged. Homologous nucleic acid sequences have a sequence identity of for example 75 to 95 %. A sequence cluster of natural occurring homologous nucleic acids such as enzymes is defined in step 1. In step 2 the nucleic acid sequences are fragmented into oligonucleotides forming the oligonucleotide pool, wherein the oligonucleotides have overlapping 5'- and/or 3'-ends. The in silico fragmented oligonucleotides are for example synthesized on a silicon platform *in vitro* (step 3), and in step 4 the oligonucleotides are reassembled randomly to form the starting nucleic acid sequence for example comprising partial changes in comparison to the original nucleic acid sequence.

### Summary of the invention

The present invention refers to a method for the production of a nucleic acid library, wherein the nucleic acid sequence is a DNA and/or RNA sequence, comprising the following steps:
a) selecting two or more nucleic acid sequences, which are for example homologous, heterologous or a combination thereof, *in silico,*
b) fragmenting of the selected nucleic acid sequences into oligonucleotides *in silico,* wherein the oligonucleotides have overlapping 5'-ends and 3'-ends which comprise the same or a different number of nucleotides,
c) producing the oligonucleotides *in vitro,*
d) mixing the oligonucleotides *in vitro,*
e) reassembling the oligonucleotides by amplifying the oligonucleotides without the use of a primer,
f) optionally amplifying the reassembled oligonucleotides with or without the use of a primer, and
g) optionally screening for the reassembled, amplified oligonucleotides of interest *in vitro* and/or *in vivo.* The screening is for example a molecular or phenotypic screening, or a combination thereof. The *in vivo* screening step takes for example place in a microorganism.

*In silico* is defined as performed on a computer or via computer simulation. *In vitro* refers to the technique of performing a given procedure in a controlled environment outside of a living organism, and *in vivo* refers to experimentation using a whole, living organism as opposed to a partial or dead organism.

If the nucleic acid sequences are homologous, the homology of the nucleic acid sequences is for example 25 to 100 %, 30 to 99 %, 35 to 98 %, 40 to 97 %, 45 to 96 %, 50 to 95 %, 55 to 90 %, 60 to 85 %, 70 to 80 %, or 75 to 95 %.

In some embodiments different oligonucleotides are produced in sub-compartments of one compartment, wherein the sub-compartments are in close spatial proximity to each other, or the oligonucleotides are synthesized on a chip.

The method of the present invention can comprise a further screening step b1) following step b) for screening of a fragmented oligonucleotide, or a further screening step e1) following step e) for screening of a reassembled oligonucleotide, or a combination of both steps b1) and e1).

The nucleic acid sequence of the present invention is for example selected from the group consisting of a gene or part thereof, a promoter, an enhancer, a regulatory element, an intron, a SNP, a transposable element, silencer, telomere, pseudogene, a repetitive sequence and combinations thereof.

The number of sequences selected in step a) of the method of the present invention are for example 2 to 1.000.000, 3 to 100.000, 4 to 10.000, 5 to 1.000, 6 to 100 or 7 to 10 sequences.

The number of oligonucleotides mixed in step d) of the present invention comprises for example 2 to 10¹², 3 to 10¹¹, 4 to 10¹⁰, 5 to 10⁹, 6 to 10⁸, 7 to 10⁷, 8 to 10⁶, 9 to 10⁵, 10 to 10⁴ or 2 to 10.000.000, 3 to 1.000.000, 4 to 100.000, 5 to 1.000, 6 to 100, or 7 to 10 oligonucleotides.

The oligonucleotides used in the method of the present invention are for example fragmented controlled based on shifting the oligonucleotides by one nucleotide along the nucleic acid sequence in one direction, which is 5'-end to 3'-end or vice versa, or based on structural coding regions, or combinations thereof.

The oligonucleotide in step e) or step f), or step e) and f) of the present invention is for example amplified and/or reassembled via polymerase chain reaction (PCR) or DNA ligase, or a combination of PCR and DNA ligase.

The present invention further refers to a method for the detection of a disease, which is for example a genetic disease, in a nucleic acid library prepared by a method according to the present invention.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### Detailed description of the invention

The present invention refers to a highly advantageous method for the production of a nucleic acid library, wherein the first steps of selecting nucleic acid sequences and fragmenting the nucleic acid sequences into oligonucleotides are performed *in silico* to save time and costs for the synthesis and fragmentation of the nucleic acid sequence *in vitro,* and to significantly increase the number of nucleic acid sequences selected for the production of the library. The nucleic acid sequences are for example homologous, heterologous or a combination thereof.

In the following, the elements of the instant invention will be described in more detail. These elements are listed with specific embodiments, however, it should be understood that they may be combined in any manner and in any number to create additional embodiments. The variously described examples and embodiments should not be construed to limit the instant invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the instant application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

The term "about" means a quantity, level, value, number, frequency, percentage, dimension, size, amount weight, length etc. that varies by as much as 10, 9, 8, 7, 6, 5, 4, 3, 2, 1 % from a reference quantity, level, value, number, frequency, percentage, dimension, size, amount weight, length etc..

"Oligonucleotide" means a linear polymer of natural or modified nucleosidic monomers linked by phosphodiester bonds or analogs thereof. The term "oligonucleotide" usually refers to a shorter polymer, e.g., comprising from about 3 to about 100 monomers, but also including 100 monomers to many thousands of monomers, e.g., 10,000 monomers, or more. Oligonucleotides may be natural or synthetic. Oligonucleotides include deoxyribonucleosides, ribonucleosides, and non-natural analogs thereof, such as anomeric forms thereof, peptide nucleic acids (PNAs), and the like, provided that they are capable of specifically binding to a target nucleic acid by way of a regular pattern of monomer-to-monomer interactions, such as Watson-Crick type of base pairing, base stacking, Hoogsteen or reverse Hoogsteen types of base pairing, or the like.

Oligonucleotides of the present invention comprise for example overlapping 5'- and 3'-ends, wherein the overlapping ends are complementary and capable of forming double strands. The overlapping ends are for example 100 % complementary or comprise mismatches and form a double strand, i.e., hybridize for example at a temperature ≤ 40 °C, e.g., at 10 °C to 40 °C, 12 °C to 38 °C, 15 °C to 36 °C, 18 °C to 34 °C, 20 °C to 32 °C, 23 °C to 30 °C, 25 °C to 28 °C, or at 10 °C, 12°C, 15 °C, 18 °C, 20 °C, 23 °C, 25 °C, 28 °C, 30 °C, 31 °C, 32 °C, 33 °C, 34 °C, 35 °C, 36 °C, 37 °C, 38 °C, 39 °C or 40 °C. The overlapping 5'- and 3'-ends of the oligonucleotide comprise the same or a different number of nucleotides. The number of nucleotides of an overlapping end is for example from 5 to several thousand or hundreds of oligonucleotides.

"Reassembled oligonucleotides" are oligonucleotides which connect via the complementary overlapping ends and an amplification reaction to form nucleic acid sequences *in vitro* and/or *in vivo* corresponding to the two or more original (*in silico*) nucleic acid sequences from which the oligonucleotides were selected by *in silico* fragmentation. Some of the nucleic acid sequences based on the reassembled oligonucleotides have or comprise a sequence which is identical to the original nucleic acid sequences and others have or comprise variations based on combinations of variations of the different original nucleic acid sequences. The reassembled oligonucleotides form the nucleic acid library.

"Primer" includes an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process are determined by the sequence of the template polynucleotide. Primers are extended by a polymerase such as a DNA polymerase. Primers usually have a length in the range of e.g., about 5 to about 500, about 10 to about 100, about 15 to about 50, about 20 to about 30, about 3 to about 40, about 5 to about 35, about 8 to about 30, about 10 to about 25, or about 12 to about 20 nucleotides.

"Tm" is used for "melting temperature", which is the temperature when a population of double-stranded nucleic acid molecules becomes half dissociated into single strands. Several equations for calculating the Tm of nucleic acids are well known in the art. As indicated by standard references, a simple estimate of the Tm value may be calculated by the equation. Tm =81.5+0.41 (% G+C), when a nucleic acid is in aqueous solution at 1 M NaCl (see e.g., Anderson and Young, "Quantitative Filter Hybridization," in Nucleic Acid Hybridization (1985) or by the assumption of 2 °C per A+T and 3 °C per G+C. Other references (e.g., Allawi, H. T. & Santa Lucia, J., Jr., Biochemistry 36, 10581-94 (1997)) include alternative methods of computation which take structural and environmental, as well as sequence characteristics into account for the calculation of Tm.

Exemplary methods for amplifying nucleic acids include the polymerase chain reaction (PCR) (see, e.g., Mullis et al. (1986) Cold Spring Harb. Symp. Quant. Biol. 51 Pt 1 :263 and Cleary et al. (2004) Nature Methods 1 :241; and U.S. Patent Nos. 4,683,195 and 4,683,202), anchor PCR, RACE PCR, ligation chain reaction (LCR) (see, e.g., Landegran et al. (1988) Science 241 : 1077-1080; and Nakazawa et al. (1994) Proc. Natl. Acad. Sci. U.S.A. 91 :360-364), self-sustained sequence replication (Guatelli et al. (1990) Proc. Natl. Acad. Sci. U.S.A. 87:1874), transcriptional amplification system (Kwoh et al. (1989) Proc. Natl: Acad. Sci. U.S.A. 86: 1173), Q- Beta eplicase (Lizardi et al. (1988) BioTechnology 6:1197), recursive PCR (Jaffe et al. (2000) J. Biol. Chem. 275:2619; and Williams et al. (2002) J Biol. Chem. 277:7790), the amplification methods described in U.S. Patent Nos. 6,391,544, 6,365,375, 6,294,323, 6,261,797, 6,124,090 and 5,612,199, isothermal amplification (e.g., rolling circle amplification (RCA), hyperbranched rolling circle amplification (HRCA), strand displacement amplification (SDA), helicase-dependent amplification (HDA), PWGA) or any other nucleic acid amplification method using techniques well known to those of skill in the art.

"Polymerase chain reaction," or "PCR," refers to a reaction for the *in vitro* amplification of specific DNA sequences by the simultaneous primer extension of complementary strands of DNA. Usually, the reaction is cycled through different temperatures optimized for each step in a thermal cycler instrument. Particular temperatures, durations at each step, and rates of change between steps depend on many factors well-known to those of ordinary skill in the art, e.g., exemplified by the references: McPherson et al., editors, PCR: A Practical Approach and PCR2: A Practical Approach (IRL Press, Oxford, 1991 and 1995, respectively). For example, in a conventional PCR using Taq DNA polymerase, a double stranded target nucleic acid may be denatured at a temperature greater than 90 °C, primers annealed at a temperature in the range 50-75 °C, and primers extended at a temperature in the range 72-78 °C.

The term "PCR" encompasses derivative forms of the reaction, including but not limited to, RT-PCR, real-time PCR, nested PCR, quantitative PCR, multiplexed PCR, assembly PCR and the like.

The method for the production of a nucleic acid library according to the present invention combines different steps performed *in silico* and *in vitro* and/or *in vivo,* respectively. In a first step nucleic acid sequences are selected *in silico.* This saves time and costs in comparison to the production of such sequences *in vitro* and/or *in vivo.* The nucleic acid sequences are DNA, RNA or combinations thereof, and the nucleic acid sequences are homologous, heterologous or combinations thereof. The nucleic acid sequences encode for example a gene or a part thereof, a promoter, an enhancer, a regulatory element, an intron, a SNP, a transposable element, silencer, telomere, pseudogene, a repetitive sequence or combinations thereof. The number of nucleic acid sequences is for example at least two up to several hundred or thousands of sequences, e.g., 10, 20, 30, 40, 50, 60, 70, 80, 90. 100, 150, 200, 250, 300, 350, 400, 500, 1000, 1500, 2000, 2500 etc.

In a next step the nucleic acid sequences selected *in silico* are fragmented into oligonucleotides *in silico* which again saves an enormous amount of time and costs in comparison to fragmentation of nucleic acid sequences *in vitro* (Fig. 1). The *in silico* fragmentation is either random, e.g., based on DNAse I patterns in the selected sequences, or controlled based on specific structures encoded by the nucleic acid sequences or design of specific oligonucleotides, or combines random and controlled fragmentation. Specific structures for controlled fragmentation are for example beta-sheet or a helix. Alternatively or in addition, controlled fragmentation is based on shifting the oligonucleotides by one nucleotide along the nucleic acid sequence in one direction, which is 5'-end to 3'-end or 3'-end to 5'-end. The *in silico* fragmentation is fast and provides easily a broad range of different oligonucleotides of the selected nucleic acid sequences.

The oligonucleotides have or comprise overlapping 5'- and 3'-ends which are complementary and allow the oligonucleotides to bind to each other for example via hybridization.

Only after the *in silico* fragmentation of the nucleic acid sequences, i.e., after the *in silico* planning of oligonucleotides, the method of the present invention turns to *in vitro* and/or *in vivo* steps. The oligonucleotides designed *in silico* are produced, i.e., synthesized *in vitro* and/or *in vivo,* e.g., according to common practice. For example the oligonucleotides are synthesized on a chip or in a compartment, such as a well of a 96 well plate, having numerous sub-compartments, such as numerous sub-wells per well of the 96 well plate (e.g., Fig. 1, step 3). These sub-compartments, such as sub-wells, are in close proximity to each other that the oligonucleotides are mixed controlled quickly and easily after their synthesis.

Once the synthesized oligonucleotides have been mixed *in vitro,* the oligonucleotides reassemble via their overlapping 5'- and 3'-ends for example by providing specific hybridization conditions such as a specific temperature, and by amplifying the oligonucleotides without or with a primer for example according to common practice including polymerase chain reaction (PCR) and/or DNAse ligase. By reassembling the oligonucleotides, the original starting nucleic acid sequences selected *in silico* are rebuilt *in vitro* and/or *in vivo.* Some of the rebuilt nucleic acid sequences based on the reassembled oligonucleotides are 100 % identical to the original starting nucleic acid sequences and others comprise variations depending on the sequence variations of the homologous and/or heterologous original starting nucleic acid sequences. These rebuilt nucleic acid sequences based on the reassembled oligonucleotides form the nucleic acid library.

Optionally the reassembled oligonucleotides are amplified with or without the use of a primer to increase the number of reassembled oligonucleotides, i.e., of rebuilt nucleic acid sequences, and optionally the reassembled, amplified oligonucleotides of interest are screened to increase for example the specificity of the nucleic acid library. The screening is for example a molecular or phenotypic screening, or a combination thereof.

The method of the present invention optionally comprises further screening steps for example *in vitro* and/or *in vivo* for example in a microorganism: For example the oligonucleotides produced by *in silico* fragmentation are screened before the synthesis of the oligonucleotides and/or the reassembled oligonucleotides are screened before the further optional amplification step to increase the number of reassembled oligonucleotides, i.e., of rebuilt nucleic acid sequences.

If the (starting) nucleic acid sequences are homologous, the homology between the nucleic acid sequences is for example 25 to 100 %, 30 to 99 %, 35 to 98 %, 40 to 97 %, 45 to 96 %, 50 to 95 %, 55 to 90 %, 60 to 85 %, 70 to 80 %, or 75 to 95 %.

The number of sequences selected in the first step of the present invention *in silico* is unlimited. It comprises for example 2 to 1.000.000, 3 to 100.000, 4 to 10.000, 5 to 1.000, 6 to 100 or 7 to 10 nucleic acid sequences. Fragmenting the nucleic acid sequences into oligonucleotides results in a high number of oligonucleotides depending on the number of nucleic acid sequences and the design of the oligonucleotides. The fragmented oligonucleotides comprise for example 2 to 10¹², 3 to 10¹¹, 4 to 10¹⁰, 5 to 10⁹, 6 to 10⁸, 7 to 10⁷, 8 to 10⁶, 9 to 10⁵, 10 to 10⁴ or 2 to 10.000.000, 3 to 1.000.000, 4 to 100.000, 5 to 1.000, 6 to 100, or 7 to 10 oligonucleotides.

The present invention further refers to a method for the detection of a disease, which is for example a genetic disease, in a nucleic acid library prepared by a method according to the present invention. Due to the high number of different nucleic acid sequences in the nucleic acid library mutations causing a disease are detectable.

## Claims

1. Method for the production of a nucleic acid library, comprising the following steps:
a) selecting two or more nucleic acid sequences *in silico,*
b) fragmenting of the selected nucleic acid sequences into oligonucleotides *in silico,* wherein the oligonucleotides have overlapping 5'-ends and 3'-ends,
c) producing the oligonucleotides *in vitro,*
d) mixing the oligonucleotides *in vitro,*
e) reassembling the oligonucleotides by amplifying the oligonucleotides without the use of a primer,
f) optionally amplifying the reassembled oligonucleotides with or without the use of a primer, and
g) optionally screening for the reassembled, amplified oligonucleotides of interest.

2. Method according to claim 1, wherein different oligonucleotides are produced in sub-compartments of one compartment, wherein the sub-compartments are in close spatial proximity to each other, or wherein the oligonucleotides are synthesized on a chip.

3. Method according to claim 1 or 2, wherein the method comprises a further screening step b1) following step b) for screening of a fragmented oligonucleotide, or a further screening step e1) following step e) for screening of a reassembled oligonucleotide, or a combination of both steps b1) and e1).

4. Method according to any one of claims 1 to claim 3, wherein the nucleic acid sequence is a DNA and/or RNA sequence.

5. Method according to any one of claims 1 to 4, wherein the nucleic acid sequence is selected from the group consisting of a gene or part thereof, a promoter, an enhancer, a regulatory element, an intron, a SNP, a transposable element, silencer, telomere, pseudogene, a repetitive sequence and combinations thereof.

6. Method according to any one of claims 1 to 5, wherein 2 to 1.000.000, 3 to 100.000, 4 to 10.000, 5 to 1.000, 6 to 100 or 7 to 10 sequences are selected in step a).

7. Method according to any one of claims 1 to 6, wherein the mixed oligonucleotides of step d) comprise 2 to 10.000.000, 3 to 1.000.000, 4 to 100.000, 5 to 1.000, 6 to 100, or 7 to 10 oligonucleotides.

8. Method according to any one of claims 1 to 7, wherein the overlapping 5'- and 3'-ends of the oligonucleotides comprise the same or a different number of nucleotides.

9. Method according to any one of claims 1 to 8, wherein the oligonucleotides are fragmented controlled based on shifting the oligonucleotides by one nucleotide along the nucleic acid sequence in one direction (5'-end to 3'-end or vice versa), or based on structural coding regions, or combinations thereof.

10. Method according to any one of claims 1 to 9, wherein the oligonucleotide in step e) or step f), or step e) and f) is amplified via polymerase chain reaction (PCR) and/or reassembled via DNA ligase, or a combination of PCR and DNA ligase.

11. Method according to any one of claims 1 to 10, wherein the screening in step g) is a molecular or phenotypic screening, or combinations thereof.

12. Method according to any one of claims 1 to 11, wherein nucleic acid sequences are homologous, heterologous or a combination thereof.

13. Method according to claim 12, wherein the homology of the nucleic acid sequences is 25 to 100 %, 30 to 99 %, 35 to 98 %, 40 to 97 %, 45 to 96 %, 50 to 95 %, 55 to 90 %, 60 to 85 %, 70 to 80 %, or 75 to 95 %.

14. Method for the detection of a disease in a nucleic acid library prepared by a method according to any one of claims 1 to 13.

15. Method of claim 14, wherein the disease is a genetic disease.
